# EUROPÄISCHE PATENTSCHRIFT

(11) **EP 2 102 178 B1**
(45) Veröffentlichungstag und Bekanntmachung des Hinweises auf die Patenterteilung: **24.11.2010**
(21) Anmeldenummer: 07847616.5
(22) Anmeldetag: 30.11.2007
(51) Int. Cl.: C07D 301/28, C08G 59/14, C07D 303/27

(54) **EPOXIDHARZZUSAMMENSETZUNGEN UND VERFAHREN ZU IHRER HERSTELLUNG**
EPOXY RESIN COMPOSITIONS AND METHOD FOR THE PRODUCTION THEREOF
COMPOSITIONS DE RÉSINE ÉPOXY ET LEUR PROCÉDÉ DE FABRICATION

(30) Priorität: 07.12.2006 EP 06125591
(43) Veröffentlichungstag der Anmeldung: 23.09.2009
(73) Patentinhaber: BASF SE, 67056 Ludwigshafen (DE)
(72) Erfinder: HENNINGSEN, Michael, 67227 Frankenthal (DE); WITTENBECHER, Lars, 68159 Mannheim (DE)
(86) Internationale Anmeldenummer: PCT/EP2007/063100
(87) Internationale Veröffentlichungsnummer: WO 2008/068205

(56) Entgegenhaltungen:
- EP-A- 0 495 339
- EP-A- 1 270 633
- WO-A-2005/061105
- DE-A1-102005 003 116
- DATABASE WPI Week 200037 Thomson Scientific, London, GB; AN 2000-426126 XP002477105 -& JP 2000 143769 A (NIPPON KAYAKU KK) 26. Mai 2000 (2000-05-26)

## Beschreibung

Die Erfindung betrifft Verfahren zur Herstellung von Epoxidharzzusammensetzungen mit niedrigem Oligomergehalt, nach dem Verfahren erhältliche Epoxidharzzusammensetzungen und deren Verwendung z. B. in licht- und witterungsstabilen Lacken und Beschichtungen bzw. Kunststoffen und in der Elektronikindustrie sowie damit hergestellte LEDs (lichtemittierende Dioden) oder optische Linsen.

Alizyklische Epoxidharzzusammensetzungen werden in vielen Bereichen der Elektronikindustrie als Beschichtungs-, Verkapselungs- oder Dichtungsmaterialien eingesetzt. Da die Zusammensetzungen wenig oder keine Aromaten enthalten, sind sie häufig licht- und vergilbungsstabil. Häufig werden die Epoxidharzzusammensetzungen ausgehend von Bisphenol-A durch Umsetzung mit Epichlorhydrin und nachfolgende Hydrierung hergestellt. Derartige Verfahren sind beispielsweise in EP-A-1 270 633 und EP-A 0 402 743 beschrieben.

Es ist auch möglich, zunächst Bisphenol-A zu hydrieren und nachfolgend mit Epichlorhydrin umzusetzen. Eine derartige Umsetzung ist beispielsweise in der EP-A-0 597 806 beschrieben.

JP-A-2000-143769 offenbart ebenfalls ein Verfahren zur Herstellung einer epoxidhaltigen Verbindung, wobei hydriertes Bisphenol-A mit flüssigem Epichlorhydrin in Gegenwart eines quartären Ammoniumsalzes und eines festen Alkalihydroxids unter Rühren bei einer Temperatur im Bereich von 20 bis 70 °C umgesetzt wird.

Während die durch Umsetzung von Bisphenol-A mit Epichlorhydrin und anschließende Hydrierung erhaltenen Produkte häufig die Anforderungen an die Produktreinheit für Elektronikanwendungen erfüllen, ist dies für die nach üblichen Verfahren hergestellten Produkte der Hydrierung von Bisphenol-A und anschließenden Umsetzung mit Epichlorhydrin nicht der Fall. Allerdings ist die Herstellung der Epoxidharzzusammensetzungen nach dem ersten Verfahren wesentlich kostenaufwendiger als nach dem letztgenannten Verfahren.

Aufgabe der vorliegenden Erfindung ist die Bereitstellung eines wirtschaftlichen Verfahrens zur Herstellung von cyclohexylgruppen-haltige Glycidylether enthaltenden Epoxidharzzusammensetzungen, die die für Elektronikanwendungen notwendigen Eigenschaften aufweisen, Insbesondere für die Herstellung von LEDs sollten die Epoxidharzzusammensetzungen farblos sein, sehr wenig oder kein (freies) Chlor enthalten und eine niedrige Viskosität wie auch einen niedrigen Nebenproduktanteil aufweisen.

Die Aufgabe wird erfindungsgemäß gelöst durch ein Verfahren zur Herstellung von cyclohexylgruppenhaltige Glycidylether enthaltenden Epoxidharzzusammensetzungen mit niedrigem Oligomergehalt durch Destillation von Zusammensetzungen, die erhältlich sind durch Kern-Hydrierung von Verbindungen der allgemeinen Formel (I) 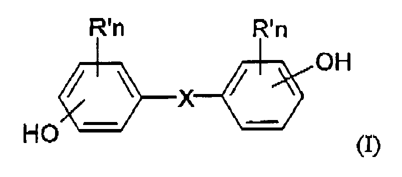 mit der Bedeutung
- -X-: -CR₂-, -CO-, -O, -S-, -SO₂-,
- R: unabhängig voneinander H, C₁₋₆-Alkyl, C₃₋₆-Cycloalkyl, in denen ein oder mehrere H durch Halogen ersetzt sein können,
- R': unabhängig voneinander C₁₋₄-Alkyl, Halogen
- n: unabhängig voneinander 0, 1, 2 oder 3
und nachfolgende Umsetzung der Hydroxylgruppen mit Epichlorhydrin,
in Dünnschichtverdampfern oder Kurzwegverdampfern bei einer Temperatur im Bereich von 150 bis 230 °C und einem Druck im Bereich von 0,005 bis 0,2 mbar.

Es wurde erfindungsgemäß gefunden, dass das Umsetzungsprodukt aus der Kern-Hydrierung von Bisphenol-A und der nachfolgenden Umsetzung mit Epichlorhydrin durch Destillation in Dünnschichtverdampfern oder Kurzwegverdampfern so aufgereinigt werden kann, dass es für Elektronikanwendungen geeignet ist. Bislang wurde eine Destillation des Umsetzungsproduktes mit Epichlorhydrin für nicht durchführbar erachtet. Insbesondere wurde aufgrund der thermischen Belastung bei der Destillation nicht davon ausgegangen, dass ein Produkt mit sehr guter Reinheit und geringem Chlorgehalt erhalten werden kann, das die Anforderungen für Elektronikanwendungen erfüllt.

Erfindungsgemäß wird die Destillation in Dünnschichtverdampfern oder Kurzwegverdampfern durchgeführt. In beiden Fällen wird eine Dünnschicht-Destillation durchgeführt, bei der durch Absenkung des Arbeitsdruckes eine erhebliche Reduzierung der Siedetemperatur erreicht wird. Beim Dünnschicht-Verdampfer wird vorzugsweise das Rohprodukt an einer Innenfläche eines von außen beheizten Rohres erhitzt, bis die leichter siedende Komponente zu verdampfen beginnt. Die Brüden werden anschließend in einem Kondensator niedergeschlagen. Beim einfachen Dünnschichtverdampfer ist dieser Kondensator möglichst dicht neben dem Verdampfer platziert, beim Kurzwegverdampfer dagegen liegt er sogar innerhalb des Verdampfermantels. Übliche Dünnschichtverdampfer können mit einem Minimaldruck von etwa 1 mbar, Kurzwegverdampfer mit einem Minimaldruck von etwa 0,001 mbar betrieben werden.

In Dünnschicht- oder Kurzwegverdampfern ist die Verweilzeit sehr kurz, Sie beträgt häufig nur wenige Sekunden.

Beim Dünnschichtverdampfer läuft das zu verdampfende Produkt an der inneren Oberfläche eines von außen beheizten zylindrischen Rohres nach unten. Der Flüssigkeitsfilm wird dabei durch ein Wischersystem ständig durchmischt, wobei eine Homogenität im zu verdampfenden Gemisch erreicht wird. Die Kondensation des Destillats erfolgt in einem außen liegenden Kondensator, an den das Vakuumsystem anschließt. Typischerweise wird der Dünnschichtverdampfer von oben mit Produkt beschickt, das an der Innenwand nach unten läuft und durch den Wischer bewegt wird. Das Destillat wird über Kopf abgezogen und im Kondensator kondensiert. Der Destillationsrückstand wird am Boden des Dünnfilmverdampfers entnommen. Durch die konstruktive Auslegung mit einer Verbindungsleitung zwischen Verdampfer und Kondensator ist der erreichbare Unterdruck auf etwa 1 mbar beschränkt.

Die Kurzweg-Destillation erlaubt es, bei möglichst niedrigen Temperaturen und kurzer Verweilzeit zu destillieren. Bei der Kurzweg-Destillation handelt es sich ebenso wie beim Dünnschichtverdampfer um einen kontinuierlichen Prozess unter vermindertem Druck.

Auch hier erfolgt die Verdampfung aus einem geheizten gewischten Film. Der im Vergleich zum Dünnschichtverdampfer deutlich niedrigere Minimaldruck im Kurzwegverdampfer wird dadurch erreicht, dass durch einen innen liegenden Kondensator die Brüden nur einen sehr kurzen Weg zurücklegen müssen. Gleichzeitig ist der Strömungsquerschnitt für die Brüden so groß wie die Verdampferfläche. Dadurch gibt es nur einen geringen Druckverlust zwischen Verdampferfläche und Kondensator. Der typische Druckbereich für die Kurzweg-Destillation ist das "Feinvakuum", d. h. von etwa 0,001 bis 1 mbar. Damit kann die erforderliche Temperatur so weit abgesenkt werden, dass es bei der kurzen Verweilzeit zu keiner thermischen Schädigung der Produkte kommt.

Durch die Anordnung des Kondensators innerhalb des Verdampfers entfällt das Brüdenrohr als Verbindungsleitung zwischen Verdampfer und Kondensator. Bei den einsetzbaren niedrigen Drücken haben die Brüden ein sehr großes Volumen, das selbst bei großen Querschnitten zu sehr hohen Geschwindigkeiten führt. Dies begrenzt beispielsweise den Druckbereich von Dünnschichtverdampfern.

Bei der Kurzweg-Destillation erfolgt vorzugsweise im Inneren eines zylindrischen Rohres die Verdampfung aus einem dünnen Film, wobei die Verdampferwand von außen über einen Doppelmantel beheizt wird. Der Film wird über ein Wischsystem ständig durchmischt, wodurch ein radialer Konzentrationsgrad weitgehend vermieden wird. Der Kondensator befindet sich innerhalb des Verdampfers. Dadurch ist der Weg, den die Dämpfe zurücklegen müssen, extrem kurz. Sofern der Abstand zwischen Verdampfer und Kondensator in der Größenordnung der mittleren freien Weglänge der Moleküle liegt, spricht man auch von Molekular-Destillation. Die mittlere freie Weglänge im Gasraum liegt dabei häufig im cm-Bereich. Erfindungsgemäß wird vorzugsweise eine derartige Molekular-Destillation durchgeführt. Die Molekular-Destillation kann auch als Kurzweg-Destillation unter Vakuum oder vermindertem Druck definiert werden.

Geeignete Dünnschichtverdampfer und Kurzwegverdampfer sind technisch erhältlich. Beispielsweise können geeignete Dünnschichtverdampfer und Kurzwegverdampfer von der VTA, Verfahrenstechnische Anlagen GmbH, D-94469 Deggendorf, bezogen werden.

Erfindungsgemäß wird bei einer Temperatur im Bereich von 150 bis 230 °C und einem Druck im Bereich von 0,005 bis 0,2 mbar destilliert.

Vorzugsweise wird eine Kurzweg-Destillation oder Molekular-Destillation durchgeführt, wobei der Druck 0,003 bis 0,3 mbar, insbesondere 0,005 bis 0,2 mbar beträgt.

Gemäß einer bevorzugten Ausführungsform wird dabei bei einer Temperatur im Bereich von 170 bis 210 °C, und einem Druck im Bereich von 0,007 bis 0,1 mbar, gearbeitet. Besonders vorteilhafte Produkte können bei Temperaturen von unterhalb 200°C, insbesondere unterhalb 190°C erhalten werden.

Die apparative Auslegung der Dünnschichtverdampfer oder Kurzwegverdampfer für die Verdampfung der erfindungsgemäß eingesetzten Zusammensetzungen erfolgt durch den Fachmann.

Das erfindungsgemäße Verfahren dient der Herstellung von Epoxidharzzusammensetzungen, die cyclohexylgruppenhaltige Glycidylether enthalten, wobei die Epoxidharzzusammensetzungen einen niedrigen Oligomergehalt aufweisen. Vorzugsweise enthalten die erfindungsgemäßen Epoxidharzzusammensetzungen sehr wenig oder keine verbleibenden Aromaten. Vorzugsweise beträgt die Höchstmenge an aromatischen Verbindungen, bezogen auf die Epoxidharzzusammensetzungen, 5 Gew.-%, insbesondere 3 Gew.-%. Besonders bevorzugt sind die Zusammensetzungen aromatenfrei.

Durch die erfindungsgemäße Destillation kann der Chlorgehalt der Zusammensetzungen stark abgesenkt werden. Üblicherweise enthalten die nach der Hydrierung von Bisphenol-A und nachfolgender Umsetzung mit Epichlorhydrin erhaltenen Epoxidharzzusammensetzungen etwa 5 Gew.-% Gesamtchlor ohne Berücksichtigung von Halogensubstituenten am Bisphenol. Die dynamische Viskosität bei 25 °C beträgt über 2000 mPas, in der Regel über 2200 mPas. Die Farbzahl (Apha) liegt üblicherweise im Bereich von 22 bis 28.

Durch das erfindungsgemäße Verfahren kann die dynamische Viskosität auf weniger als 2000 mPas, vorzugsweise auf weniger als 1200 mPas (bei 25 °C) verringert werden. Der Chlorgehalt (Gesamtchlor) kann auf niedrige Werte von maximal 3 Gew.-%, vorzugsweise 2,5 Gew.-% und weniger, insbesondere 1,5 Gew.-% und weniger vermindert werden. Beispielsweise kann die Verminderung von 4,9 Gew.-% auf 1,3 Gew.-% erfolgen. Der Gesamtchlorgehalt wird dabei bestimmt mit der Methode nach Schoeniger und beinhaltet nicht mögliche Halogensubstituenten in R und R', die bevorzugt jedoch nicht vorliegen.

Die dynamische Viskosität kann bevorzugt auf 1000 bis 1300 mPas, insbesondere 1100 bis 1200 mPas vermindert werden. Damit führt die Kurzwegverdampfung zu Destillaten mit geringerer Viskosität und geringerem Chlorgehalt.

Bei der erfindungsgemäßen Destillation beträgt der Destillatanteil vorzugsweise 25 bis 90 Gew.-%, besonders bevorzugt 40 bis 90 Gew.-%, insbesondere 55 bis 86 Gew.-%. Der Destillatanteil kann dabei durch entsprechenden Betrieb des Kurzwegverdampfers bzw. Dünnschichtverdampfers eingestellt werden.

Im erfindungsgemäßen Verfahren werden Zusammensetzungen destilliert, die erhältlich sind durch Kern-Hydrierung von Verbindungen der allgemeinen Formel (I) 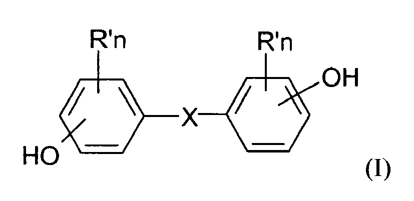 mit der Bedeutung
- -X-: -CR₂-, -CO-, -O, -S-, -SO₂-,
- R: unabhängig voneinander H, C₁₋₆-Alkyl, C₃₋₆-Cycloalkyl, in denen ein oder mehrere H durch Halogen ersetzt sein können,
- R': unabhängig voneinander C₁₋₄-Alkyl, Halogen
- n: unabhängig voneinander 0, 1, 2 oder 3
und nachfolgende Umsetzung der Hydroxylgruppen mit Epichlorhydrin.

Die Verbindung der allgemeinen Formel (I) ist vorzugsweise ausgewählt aus solchen Verbindungen, in denen -X- -CR₂-, -CO-, oder -O- bedeutet. R bedeutet dabei vorzugsweise H oder C₁₋₃-Alkyl, wobei ein oder mehrere H durch Halogen ersetzt sein können. Halogen ist dabei vorzugsweise Fluor. n hat vorzugsweise den Wert 0, und in R' bedeutet Halogen Fluor.

Besonders bevorzugt bedeutet in der Verbindung der allgemeinen Formel (I) X -CH₂-, - C(CH₃)₂-, -C(CF₃)₂-, -CO- oder -O-, n hat den Wert 0, und die Hydroxylgruppen stehen jeweils in p-Stellung. Besonders bevorzugt eingesetzte Verbindungen der allgemeinen Formel (I) sind Bisphenol-A und Bisphenol-F.

Die Hydrierung der Verbindungen kann dabei nach bekannten Verfahren erfolgen. Für geeignete Verfahrensweisen und Katalysatoren kann beispielsweise auf JP-A-2000-143769 und EP-A-0 597 806 verwiesen werden. Geeignete Katalysatoren sind ferner in EP-A-0 402 743, EP-A-1 270 633, US 6,060,611, EP-A-0 814 098 beschrieben. Geeignete Hydrier-Katalysatoren sind zudem in US 5,530,147, JP-A-2003-12659, US 6,756,453, WO 2004/009526, JP-A-2002-249488, JP-A-2002-338559, JP-A-2002-338561 und WO 03/103830 beschrieben. Für typische Grundstrukturen der cyclohexylgruppen-haltigen Glycidylether kann auf EP-A-1 270 633, EP-A-0 597 806 und JP-A-2000-143769 verwiesen werden. Dabei ist zu beachten, dass die Produktzusammensetzung für die Hydrierung von Bisphenol-A und nachfolgende Umsetzung mit Epichlorhydrin eine andere ist als für die Umsetzung von Bisphenol-A mit Epichlorhydrin und nachfolgender Destillation. Durch Hydrierung von Bisphenol-A und nachfolgende Umsetzung mit Epichlorhydrin erhaltene Epoxidharze sind beispielsweise von Leuna Harze unter der Bezeichnung Epilox®P2200 erhältlich. Ähnliche Produkte sind von Hexion unter der Bezeichnung Eponex®1510 erhältlich. Das Produkt Eponex 1510 von Hexion weist beispielsweise einen Epoxid-Äquivalentwert von 213 g/Äq., einen Gesamtchlorgehalt von 4,9 %, einer Farbzahl von 23 Apha, eine dynamische Viskosität bei 25 °C von 2290 mPas, eine kinematische Viskosität bei 25 °C von 2100 mm²/s und eine Dichte bei 25 °C von 1,090 g/ml auf.

Dieses Ausgangsgemisch wird erfindungsgemäß so destilliert, dass der Destillatanteil 25 bis 90 Gew.-%, vorzugsweise 55 bis 86 Gew.-% beträgt. Im technischen Verfahren wird insbesondere bei Temperaturen von 207 bis 240 °C und einem Druck von 0,02 bis 0,13 mbar gearbeitet. Hierbei ist eine Verminderung des Chlorgehalts von 4,9 % auf 1,3 % und eine Verminderung der dynamischen Viskosität von 2290 mPas auf 1100 bis 1200 mPas möglich.

Die Destillate enthalten insbesondere einen deutlich niedrigeren Oligomeren-Anteil als die Ausgangsgemische.

Sie sind farblos, enthalten wenig oder kein Chlor, zeigen eine niedrige Viskosität und weisen einen niedrigen Nebenprodukt-Anteil auf. Damit sind sie besonders vorteilhaft in Beschichtungs-, Verkapselungs- und Dichtungsmaterialien für die Elektronikindustrie einzusetzen. Besonders bevorzugt können sie zur Verkapselung von LEDs eingesetzt werden. Derartige Anwendungen sind beispielsweise auch in JP-A-2000-143769, EP-A-0 597 806 und insbesondere EP-A-1 270 633 und US 6,060,611 beschrieben. Durch die niedrige Viskosität sind sie insbesondere gut in diesen Anwendungen verarbeitbar, so dass blasenfreie und vollständige Beschichtungen und Verkapselungen möglich sind. Durch die Durchsichtigkeit, insbesondere Farblosigkeit sind sie sehr gut für optische Anwendungen geeignet. Durch den sehr geringen Chlorgehalt wird eine Oxidation metallischer Komponenten zuverlässig verhindert.

Die Erfindung betrifft auch die Epoxidharzzusammensetzungen mit niedrigem Oligomergehalt, die nach dem vorstehenden Verfahren erhältlich sind. Diese Zusammensetzungen werden nachfolgend noch näher charakterisiert. Die Epoxidharzzusammensetzungen werden dabei durch die Sequenz Hydrierung der Bisphenol-Verbindungen, nachfolgende Umsetzung der Hydroxylgruppen mit Epichlorhydrin und nachfolgende Kurzwegdestillation hergestellt.

Bevorzugt handelt es sich bei den erfindungsgemäß hergestellten Epoxidharzzusammensetzungen um solche aus einem Verfahren zur Herstellung von cyclohexylgruppenhaltige Glycidylether enthaltenden Epoxidharzzusammensetzungen mit niedrigem Oligomergehalt durch Destillation von Zusammensetzungen, die erhältlich sind durch Hydrierung von Verbindungen der allgemeinen Formel (I) und nachfolgende Umsetzung der Hydroxylgruppen mit Epichlorhydrin sowie nachfolgende Kurzwegverdampfung.

Dabei beträgt der Anteil von Verbindungen mit dem 0,5 bis 1,12-fachen des Molekulargewichts vorzugsweise mindestens 70 Gew.-%, insbesondere mindestens 80 Gew.-%, während der Anteil von Verbindungen mit dem 1,12 bis 1,53-fachen des Molekulargewichts vorzugsweise weniger als 20 Gew.-%, vorzugsweise weniger als 15 Gew.-% beträgt. Der Anteil von Verbindungen mit dem 1,53 bis 2,53-fachen des Molekulargewichts der Verbindung der allgemeinen Formel (II) beträgt vorzugsweise weniger als 1,5 Gew.-%, insbesondere weniger als 0,5 Gew.-%. Diese Werte gelten insbesondere bei einer Manteltemperatur von 170 bis 210 °C und einem Druck von 0,007 bis 0,01 mbar. Bei einer Umsetzung in technischem Maßstab bei Manteltemperaturen von 207 bis 238 °C und einem Druck im Bereich von 0,03 bis 0,11 mbar beträgt der Anteil von Verbindungen mit dem 0,5 bis 1,12-fachen des Molekulargewichts der Verbindung der allgemeinen Formel (II) besonders bevorzugt mindestens 70 Gew.-%, der Anteil von Verbindungen mit dem 1,12 bis 1,53-fachen des Molekulargewichts der Verbindung der allgemeinen Formel (II) vorzugsweise weniger als 26 Gew.-% und der Anteil von Verbindungen mit dem 1,53 bis 2,53-fachen des Molekulargewichts der Verbindung der allgemeinen Formel (II) weniger als 2,3 Gew.-%.

Für den Bisglycidylether des hydrierten Bisphenol-A ergibt sich bei einem Molekulargewicht von 340 g/mol eine Aufteilung in entsprechende Bereiche von 180 bis 380 g/mol, 380 bis 520 g/mol und 520 bis 860 g/mol.

Die Gewichtsverteilung der Zusammensetzung wird dabei durch Gelpermeationschromatographie bestimmt. Dabei werden folgende Bedingungen eingehalten:

### GPC-Meßbedingungen

| | |
|---|---|
| Stationäre Phase: | 5 Styroldivinylbenzolgelsäulen PSS SDV [1xE2Å//3xE4Å//1E5Å] (je 300x8 mm) von PSS GmbH (Temperierung: 35 °C) |
| | |
| Mobile Phase: | THF (Fluß: 1,2 ml/min) |
| | |
| Eichung: | MG 500-5 000 000 g/mol mit PS-Eichkit von Polymer Laboratories Im Oligomerbereich: Ethylbenzol / 1,3-Diphenylbutan / 1,3,5-Triphenylhexan / 1,3,5,7-Tetraphenyloktan / 1,3,5,7,9-Pentaphenyldekan Auswertegrenze: 180 g/mol |
| | |
| Detektion: | RI (Brechungsindex) Waters 410 UV (bei 254 nm) Spectra Series UV 100 |

Das zur Destillation eingesetzte Gemisch, beispielsweise Eponex® 1510, zeigt im GPC-Diagramm zwei starke Peaks im Bereich von 400 bis 500 g/mol und im Bereich von 520 bis 700 g/mol. Nach der Destillation ist der Peak im Bereich von 520 bis 700 g/mol wesentlich kleiner, häufig maximal ein Drittel der Höhe des Peaks im Bereich von 400 bis 500 g/mol. Dafür ist ein wesentlich vergrößerter Peak im Bereich von 300 bis 400 g/mol enthalten.

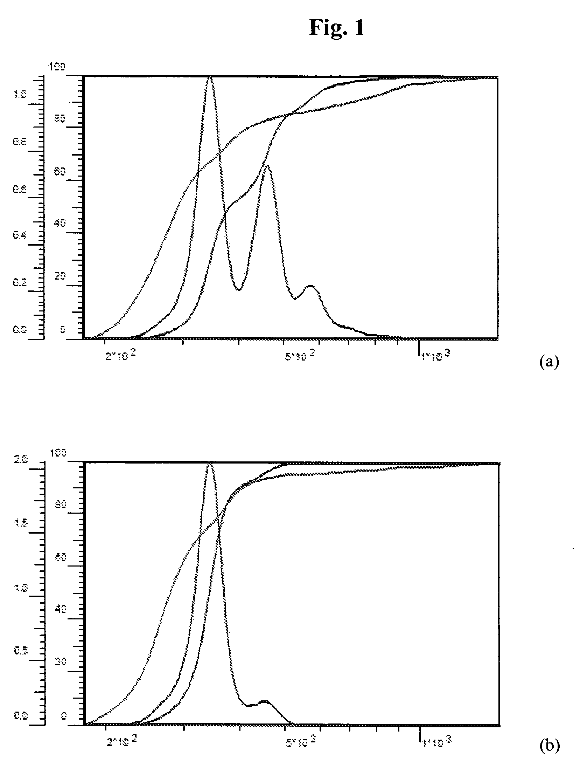

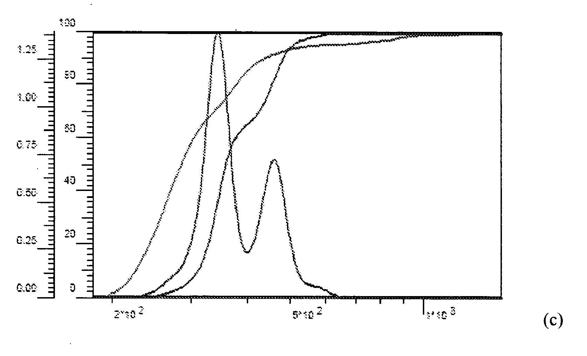

Figur 1 zeigt GPC-Verteilungen für den Einsatzstoff (a) und zwei Destillate (b, c).

Der Hauptunterschied des Produkts nach der Destillation zum Produkt vor der Destillation liegt darin, dass der ausgeprägte Peak im Bereich von 520 bis 700 g/mol zu einer kleinen Schulter und damit nicht mehr zu einem individuellen Peak wird.

Während die Hauptprodukte die gleichen sind, ändert sich das Nebenprodukt-Spektrum. Die spezifischen Nebenprodukte stellen quasi einen Fingerabdruck des Verfahrens dar und unterscheiden die Produkte von denen nach bisherigen Verfahren erhaltenen Produkten, insbesondere auch gegenüber durch zunächst Umsetzung mit Epichlorhydrin und nachfolgender Hydrierung erhaltenen Produkten. Durch das vorteilhafte Eigenschaftsspektrum eignen sich die erfindungsgemäßen Zusammensetzungen für alle Anwendungen in Lacken und in der Elektronikindustrie, beispielsweise Beschichtungs-, Verkapselungs-, Guss- oder Dichtungsmassen. Die Zusammensetzungen sind lichtstabil, insbesondere UV-stabil und neigen nicht zum Vergilben und weisen eine geringe oder keine Eigenfärbung auf.

Die Erfindung betrifft auch eine LED mit einem Dichtungs- und/oder Verkapselungsmaterial, das eine Epoxidharzzusammensetzung, gegebenenfalls in gehärteter Form, wie sie vor vorstehend beschrieben ist, enthält oder daraus besteht. Zudem betrifft die Erfindung optische Linsen aus einem entsprechenden Material.

Die Erfindung wird durch die nachstehenden Beispiele näher erläutert.

### Beispiel 1

Als Einsatzstoff wurde der hydrierte Bisglycidylether Eponex® 1510 von Hexion eingesetzt, der auf hydriertem Bisphenol-A basiert. Die Destillation wurde als Kurzweg-Destillation bzw. Molekular-Destillation bei Temperaturen im Bereich von 170 bis 210 °C und Drücken im Bereich von 0,007 bis 0,01 mbar durchgeführt.

Bei einer Manteltemperatur von 170 °C und einem Druck von 0,01 mbar sowie einem Destillatanteil von 53,2 % wurde der Produktanteil im Bereich von 180 bis 380 g/mol von 50,42 % auf 86,62 % erhöht, während der Bereich von 380 bis 520 g/mol von 35,65 % auf 13,01 %, der Bereich von 520 bis 860 g/mol von 13,15 % auf 0,38 % und der Bereich von 860 bis 1500 g/mol von 0,74 auf 0,02 % vermindert wurden.

Im Einzelnen wurden folgende Ergebnisse erhalten (GPC-Analyse wie vorstehend beschrieben):

| | Manteltemp. [°C] | Druck [mbar] | Zulauf [g/h] | Verhältnis Destillat/ Rückstand [%] | Farbzahl [Apha] | EEW [g/eq] | Gesamt-Chlor [%] | dyn Viskosität 25 °C mPa*s | kin. Viskosität 25 °C mm2/s | kin. Viskosität 25 °C mm2/s | GPC (g/mol) | | | |
|---|---|---|---|---|---|---|---|---|---|---|---|---|---|---|
| | | | | | | | | | | | 180-380 | 380-520 | 520-860 | 860-1500 |
| | | | | | | | | | | | rel.% | rel.% | rel.% | rel.% |
| Einsatz | | | | | 23-28 | 213 | 4,9 | 2290 | 2100 | 2100 | 50,42 | 35,65 | 13,15 | 0,74 |
| Destillat | 170 | 0,01 | 633 | 46,8 | | 197 | 1,3 | 1130 | 1060 | 1060 | 86,62 | 13,01 | 0,38 | 0,02 |
| Destillat | 200 | 0,007 | 467 | 16,6 | 13 | 204 | 3,3 | 1770 | 1640 | | 61,53 | 35,06 | 3,37 | 0.03 |

Bei höherer Destillationstemperatur steigen Gesamtchlor und Viskosität an. In der Figur 1 sind die Molmassenverteilungen (D) für den Einsatzstoff (a), das Destillat bei 170°C (b) und das Destillat bei 200°C (c) angegeben. Als Integral sind rel. W (log M) [ ] angegeben.

## Patentansprüche

1. Verfahren zur Herstellung von cyclohexylgruppenhaltige Glycidylether enthaltenden Epoxidharzzusammensetzungen mit niedrigem Oligomergehalt durch Destillation von Zusammensetzungen, die hergestellt werden durch Kern-Hydrierung von Verbindungen der allgemeinen Formel (I) 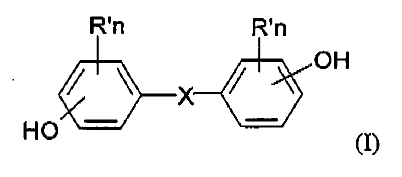 mit der Bedeutung
-X- -CR₂-, -CO-, -O, -S-, -SO₂-,
R unabhängig voneinander H, C₁₋₆-Alkyl, C₃₋₆-Cycloalkyl, in denen ein oder mehrere H durch Halogen ersetzt sein können,
R' unabhängig voneinander C₁₋₄-Alkyl, Halogen
n unabhängig voneinander 0, 1, 2 oder 3
und nachfolgende Umsetzung der Hydroxylgruppen mit Epichlorhydrin,
in Kurzwegverdampfern bei einer Temperatur im Bereich von 150 bis 230 °C unter einem Druck im Bereich von 0,005 bis 0,2 mbar.

2. Verfahren nach Anspruch 1, **dadurch gekennzeichnet, dass** in der Verbindung der allgemeinen Formel (I) X -CH₂-, -C(CH₃)₂-, -C(CF₃)₂-, -CO- oder -O- bedeutet, n den Wert 0 hat und die Hydroxylgruppen jeweils in p-Stellung stehen.

3. Verfahren nach einem der Ansprüche 1 oder 2, **dadurch gekennzeichnet, dass** der Destillatanteil 25 bis 90 Gew.-%, bezogen auf die zur Destillation eingesetzten Zusammensetzungen, beträgt.

## Claims

1. A process for preparing epoxy resin compositions which comprise glycidyl ethers comprising cyclohexyl groups and have a low oligomer content by distillation of compositions which are prepared by ring hydrogenation of compounds of the general formula (I) 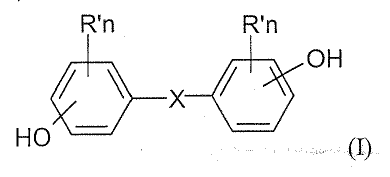 where
-X- is -CR₂-, -CO-, -O, -S-, -SO₂-,
the radicals R are each, independently of one another, H, C₁₋₆-alkyl, C₃₋₆-cycloalkyl, in which one or more H atoms can be replaced by halogen,
the radicals R' are each, independently of one another, C₁₋₄-alkyl, halogen,
the indices n are each, independently of one another, 0, 1, 2 or 3,
and subsequent reaction of the hydroxyl groups with epichlorohydrin,
in short path evaporators at a temperature in the range from 150 to 230°C under a pressure in the range from 0.005 to 0.2 mbar.

2. The process according to claim 1, wherein, in the compound of the general formula (I), X is -CH₂-, -C(CH₃)₂-, -C(CF₃)₂-, -CO- or -O-, n is 0 and the hydroxyl groups are in the p position relative to one another.

3. The process according to either of claims 1 and 2, wherein the proportion of distillate is from 25 to 90% by weight, based on the compositions used for the distillation.

## Revendications

1. Procédé pour la préparation de compositions de résine époxyde contenant des glycidyléthers contenant des groupes cyclohexyle présentant une faible teneur en oligomères par distillation de compositions qui ont été préparées par hydrogénation de noyau de composés de formule générale (I) 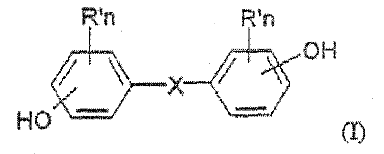 où
-X- signifie -CR₂-, -CO-, -O, -S-, -SO₂-,
R indépendamment les uns des autres, signifient H, C₁₋₆-alkyle, C₃₋₆-cycloalkyle, où un ou plusieurs H peuvent être remplacés par halogène,
R' signifient, indépendamment les uns des autres C₁₋₄- alkyle, halogène,
n indépendamment les uns des autres, valent 0, 1, 2 ou 3
et transformation consécutive des groupes hydroxyle avec de l'épichlorhydrine,
dans des évaporateurs instantanés à une température dans la plage de 150°C à 230°C et à une pression dans la plage de 0,005 à 0,2 mbar.

2. Procédé selon la revendication 1, **caractérisé en ce que** dans le composé de formule générale (I) X signifie -CH₂-, -C(CH₃)₂-, -C(CF₃)₂-, -CO- ou -O-, n vaut 0 et les groupes hydroxyle se trouvent à chaque fois en position para.

3. Procédé selon l'une quelconque des revendications 1 ou 2, **caractérisé en ce que** la proportion de distillat représente 25 à 90% en poids, par rapport aux compositions utilisées pour la distillation.
